(19) Europäisches Patentamt
European Patent Office
Office européen des brevets

(11) **EP 2 520 277 A1**

(12) **EUROPEAN PATENT APPLICATION**
published in accordance with Art. 153(4) EPC

(43) Date of publication:
**07.11.2012 Bulletin 2012/45**

(51) Int Cl.:
**A61K 8/81** *(2006.01)*    **A61K 8/02** *(2006.01)*
**A61K 8/41** *(2006.01)*    **A61Q 19/00** *(2006.01)*

(21) Application number: **10840994.7**

(22) Date of filing: **27.12.2010**

(86) International application number:
**PCT/JP2010/073574**

(87) International publication number:
**WO 2011/081138 (07.07.2011 Gazette 2011/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.12.2009 JP 2009298191**

(71) Applicant: **Kao Corporation
Chuo-ku
Tokyo 103-8210 (JP)**

(72) Inventor: **OKUBO, Koji
Tokyo 131-8501 (JP)**

(74) Representative: **HOFFMANN EITLE
Patent- und Rechtsanwälte
Arabellastrasse 4
81925 München (DE)**

(54) **AQUEOUS COSMETIC PREPARATION**

(57)    A method for producing an aqueous cosmetic composition containing the following components (A), (B) and (C):
(A) a primary amine, a secondary amine, a tertiary amine or a quaternary amine having 12 to 60 carbons in total,
(B) an anionic polymer having a carboxyl group, and
(C) water, and, containing: a liquid crystal constituted by the components (A), (B) and (C); obtained by mixing the components (A), (B) and (C); heating the mixture to a temperature equal to or higher than the melting point of the component (A) to homogenize the mixture; and neutralizing the component (B) with the component (A) to form a salt.

EP 2 520 277 A1

**Description**

FIELD OF THE INVENTION

The present invention relates to an aqueous cosmetic composition.

BACKGROUND OF THE INVENTION

The barrier function of the skin is very important for the health of the skin, and it has been known that the transdermal transpiration amount of moisture of the skin increases in atopy and rough skin. It has been known that solid fats such as vaseline and wax have a high effect as agents for enhancing barrier function. However, although vaseline and wax have a high occluding effect, they have tackiness, and thus considerations for improving feel upon use when they are used have been done.
For example, an emulsion-type skin composition containing an oil component in which 90% by weight or more of the whole amount is composed of vaseline and having an internal oil phase particle size of 500 nm or less (Patent Document 1), a skin external composition containing white vaseline, a cosmetic powder, a phosphorylcholine-like group-containing polymer, an anti-inflammatory agent or an antibacterial agent (Patent Document 2), and the like have been suggested.

However, tackiness could not be sufficiently suppressed by these compositions, and thus it was difficult to achieve a balance between a fine feeling upon use and a moisture retention effect.

PRIOR ART DOCUMENTS

PATENT DOCUMENT

Patent Document 1: JP-A-2001-72581
Patent Document 2: JP-A-2003-26608

SUMMARY OF THE INVENTION

The present invention provides a method for producing an aqueous cosmetic composition containing the following components (A), (B) and (C):

(A) a primary amine, a secondary amine, a tertiary amine or a quaternary amine having 12 to 60 carbons in total,
(B) an anionic polymer having a carboxyl group, and
(C) water, and

being characterized by containing a liquid crystal constituted by the components (A), (B) and (C),
wherein the method includes:

mixing the components (A), (B) and (C);
heating the mixture to a temperature equal to or higher than the melting point of the component (A) to homogenize the mixture; and
neutralizing the component (B) with the component (A) to form a salt.

Furthermore, the present invention provides an aqueous cosmetic composition obtained by the production method.

EFFECTS OF THE INVENTION

The aqueous cosmetic composition of the present invention has no stickiness, provides a fine feeling upon use, has a high occluding effect and is excellent in moisture retention effect.

DESCRIPTION OF EMBODIMENTS

The present invention relates to a cosmetic composition that has no stickiness, provides a fine feeling upon use and is excellent in occluding effect.

The present inventor has found that a polymer salt obtained by combining a specific amine and an anionic polymer having a carboxyl group forms a polymer liquid crystal (hereinafter described as a liquid crystal) by being mixed with water, and an aqueous cosmetic composition that has no stickiness, provides a fine feeling upon use, has a high occluding effect and is excellent in moisture retention effect can be obtained by dispersing the liquid crystal in a gel or water.

The amine of the component (A) used in the present invention has 12 to 60 carbons in total, preferably 14 to 36 carbons in total and has a saturated or unsaturated hydrocarbon group of a straight chain or branched chain, and may also have an amide group, a hydroxyl group, an ether bond and the like. In addition, it is preferable that the component (A) is free from so-called a ceramide in which an aliphatic acid and sphingosine are linked via an amide bond. The ceramide as used herein means natural-type ceramides such as ceramide NDS, ceramide NS, ceramide NP, ceramide NH, ceramide AS, ceramide AH, ceramide AP, ceramide EOS, ceramide EOH and ceramide EOP.

The amine of the component (A) preferably has an alkyl group or an alkenyl group having 14 to 36 carbons, and examples thereof may include an alkyl group such as a lauryl group, a myristyl group, a palmityl group, an isopalmityl group, a stearyl group and an isostearyl group; an alkenyl group such as an oleyl group, a linolic group and a palmitoleyl group; and the like.

More specifically, the primary amine may include a sphingosine such as laurylamine, stearylamine, oleylamine, sphingosine and phytosphingosine, and the like; the secondary amine may include distearylamine, hydroxyethylisostearyloxyisopropanolamine, hydroxyethylstearyloxyisopropanolamine and the like; the tertiary amine may include dimethylstearylamine and the like; and the quaternary amine may include stearyltrimethylammonium chloride and the like. Among these, the primary amine and secondary amine are preferable, and those having a branched alkyl chain are more preferable.

One or more of the component (A) may be used, and the component (A) is preferably contained by from 0.01 to 40% by weight, more preferably by from 0.1 to 20% by weight in the whole composition in view of that the anionic polymer having a carboxyl group that is the component (B) mentioned below can be neutralized sufficiently.

The component (B) used in the present invention is an anionic polymer having a carboxyl group. Specifically, a polymer obtained by polymerizing a monomer having a carboxyl group such as acrylic acid and methacrylic acid is preferable. Furthermore, the component (B) may be a polymer obtained by copolymerization of a monomer such as acrylic acid and methacrylic acid and other monomer that is hydrophobic and has a polymerizable group such as an unsaturated bond. Furthermore, the component (B) may contain a crosslinking agent.

As the component (B), an anionic polymer containing acrylic acid or methacrylic acid as a monomer is preferable.

More specific examples may include a polyacrylic acid, a polymethacrylic acid, an acrylic acid-alkyl methacrylate copolymer, an acrylates/alkyl acrylate (C10-30) cross polymer, a carboxyvinyl polymer (Carbomer), a (meth) acrylic acid/ alkyl (meth) acrylate (C10-30)/(meth)acrylic acid polyoxyethylene alkyl (C10-30) ether and the like.

As these polymers, commercial products such as ACULYN 88, ACULYN 22, ACULYN 28 and ACULYN 38 (these are manufactured by Rohm & Haas), and CARBOPOL 980, CARBOPOL 981, CARBOPOL Ultrez 10, CARBOPOL Ultrez 20, CARBOPOL Ultrez 21, CARBOPOL ETD2020, CARBOPOL AQUA-SF1, PEMULEN TR-1 and PEMULEN TR-2 (these are manufactured by Lubrizol Advanced Materials) may be used.

Furthermore, as the component (B), polymer polysaccharides having a carboxyl group may also be used. Examples of such polymer polysaccharides having a carboxyl group may include pectin, hyaluronic acid, gellan gum and the like.

Among these, pectin is preferable in view of that it is readily neutralized with an alkylamine.

The anionic polymer of the component (B) preferably has a weight average molecular weight of from 100,000, more preferably from 1,000,000, to 10,000,000, since easiness of handling and a sufficient occluding effect by a liquid crystal can be obtained.

One or more of the component (B) may be used, and is preferably incorporated in the whole composition by from 0.01 to 20% by weight, more preferably by from 0.1 to 10% by weight, from the viewpoint that the liquid crystal made by the component (A), component (B) and component (C) can be contained sufficiently in the cosmetic composition and thus high moisture retention property can be obtained.

In the present invention, the weight ratio of the component (A) and the component (B) is preferably (A)/(B)$\geq$1, more preferably from 1 to 90, in view of the formation of the liquid crystal.

The water of the component (C) used in the present invention is preferably contained in the whole composition by 10% by weight or more, further by from 50 to 99.8% by weight.

The component (B) that has been neutralized by the component (A) forms a liquid crystal by being mixed with a part or entirety of the component (C), and becomes a gel form. The cosmetic composition of the present invention shows this gel form or a water dispersion of the gel. This composition in which the liquid crystal has been formed can form a film that extends smoothly and has high occluding property on the skin. During use, stickiness is suppressed, refreshing feeling of use is provided, and dry feeling can be reduced.

The aqueous cosmetic composition of the present invention may further contain (D) an oil agent that is in a liquid form at 25°C.

Such oil agent is not limited as long as it is used for general cosmetic compositions, and examples may include hydrocarbon oils such as liquid paraffin, liquid isoparaffin, hydrogenated polyisobutene, squalene, n-octane, n-heptane and cyclohexane; ester oils such as diisostearyl maleate, octyldodecyl lactate, isotridecyl isononanoate, octyldodecyl myristate, isopropyl palmitate, isopropyl isostearate, butyl stearate, myristyl myristate, isopropyl myristate, octyldodecyl myristate, di-2-ethylhexyl adipate, diisopropyl sebacate, neopentyl glycol dicaprate, tricaproin, olive oil, meadowfoam oil and pentaerythrityl 2 ethylhexanoate; and ether oils such as cetyl isobutyl ether, dioctyl ether, ethylene glycol monolauryl ether, ethylene glycol dioctyl ether and glycerol monooleyl ether.

One or more of the component (D) may be used, and is preferably contained in the whole composition by from 0.01 to 50% by weight, further by from 0.1 to 30% by weight, in view of that the moisture retention effect of the obtained liquid crystal is enhanced and feeling upon use is adjusted.

The aqueous cosmetic composition of the present invention can further contain, in addition to the above-mentioned components, components used in general cosmetic compositions such as a surfactant, an alcohol, a polyol, a polymer compound, an ultraviolet absorber, an antioxidant, a powdery, a dye, a fragrance, a pigment, an antifouling agent, a moisture retention agent and the like, and can be formed into a cosmetic composition used for the skin or hair.

The aqueous cosmetic composition of the present invention may be produced by forming a liquid crystal constituted by the components (A), (B) and (C) by mixing the components (A), (B) and (C), heating the mixture to a temperature equal to or higher than the melting point of the component (A) to homogenize the mixture, and neutralizing the component (B) with the component (A) to form a salt.

Furthermore, the aqueous cosmetic composition of the present invention can be produced by mixing the component (A), component (B) and a part of the component (C), heating the mixture to a temperature equal to or higher than the melting point of the component (A) and neutralizing the component (B) with the component (A) to form a salt, thereby forming a liquid crystal constituted by the components (A), (B) and (C), followed by adding the residual water thereto while stirring to uniformly disperse the liquid crystal in the water.

In the case when the oil agent that is in a liquid form at 25°C (D) is further incorporated in the aqueous cosmetic composition, the component (A), component (B) and a part of the component (C) is mixed and heated to a temperature equal to or higher than the melting point of the component (A), the component (B) is neutralized with the component (A) to form a salt, and thereafter a liquid crystal constituted by the components (A), (B) and (C) is formed. Thereafter the liquid crystal is homogenized by mixing with the component (D), and the residual water is further added thereto, thereby the aqueous cosmetic composition of the present invention can be produced.

In the case when the water is added in portions, when a part of water that is added initially is defined as (C'), the weight ratio of the components (A), (B) and (C') is preferably (C')/((A)+(B))=0.01 or more, more preferably from 0.01 to 0.5, in view of the formation of the liquid crystal.

In either case, the liquid crystal constituted by the components (A), (B) and (C) is formed by adding (at least a part of) water to the mixture of the component (A) and component (B). It can be confirmed that the liquid crystal has been formed by representing anisotropy by a polarization microscope, and more specifically, can be confirmed by detecting a structure on an amorphous by a broad angle X-ray scattering analysis.

In the present invention, since the liquid crystal forms a thin film of the liquid crystal on the skin in a drying process to suppress moisture transpiration from the skin, a more excellent moisture retention effect can be obtained.

The aqueous cosmetic composition of the present invention can be applied as a skin lotion, a beauty essence, a milky lotion, a hair cosmetic composition or the like.

In the aqueous cosmetic composition of the present invention, it is more preferable to use the preferable scopes of the respective components and of the respective production methods in combination.

EXAMPLES

Examples 1 to 11 and Comparative Examples 1 to 2

The aqueous cosmetic compositions having the compositions shown in Table 1 were produced, and the formation of a liquid crystal, occluding effect, feeling upon use and moisture retention effect were evaluated. The results are shown collectively in Table 1.

(Production Method)

The component (A) and component (B) were mixed homogeneously at 80°C, water 1 that had been heated to 80°C was added thereto, and the mixture was further mixed. Furthermore, water 2 was added thereto to disperse the mixture in the water 2, thereby an aqueous cosmetic composition was obtained.

(Evaluation method)

(1) Formation of liquid crystal:

Each cosmetic composition was observed by showing anisotropy by a polarization microscope and by detecting a structure on an amorphous by a broad angle X-ray scattering analysis.
The cosmetic composition in which a liquid crystal was formed is represented by "O", and the cosmetic composition in which no liquid crystal was formed is represented by "×".

(2) Occluding effect:

20.00 g of water is put into a 50 mL vial bottle (Pierce Vial CV-400, manufactured by As One Corporation). 0.05 g of purified water or each cosmetic composition was applied uniformly to a filter paper having a diameter of about 2 cm (ADVANTEC filter paper 5C, manufactured by Toyo Roshi Kaisha, Ltd.), and the filter paper was left for 30 minutes and put on the opening of the vial bottle with the applied surface facing up. The rubber attached to the lid was removed, the screen was removed, thereafter the lid was put on the vial bottle to fix the filter paper. Thereafter the vial bottle was stored in a room at 20°C under a humidity of 40% for 24 hours, and an amount of moisture transpiration was obtained by measuring the weights before and after the storage. The degree of the suppression of the vaporization of moisture by the cosmetic composition as compared to the case when water was applied alone was obtained by calculating an occluding effect according to the following Formulas 1 to 3.

$$\text{Change in weight during application of water = initial weight - weight at after 24 hours} \quad \text{(Formula 1)}$$

$$\text{Change in weight during application of sample = initial weight - weight at after 24 hours} \quad \text{(Formula 2)}$$

$$\text{Occluding effect (\%) = (1-(Formula 2)/(Formula 1))} \times 100 \quad \text{(Formula 3)}$$

(3) Feeling upon use (absence of stickiness):

The absence of stickiness when each cosmetic composition was used was evaluated by sensory evaluation by ten specialized panel members according to the following criteria, and an average score was obtained.

5; The composition has considerably little stickiness.
4; The composition has little stickiness.
3; Cannot say either.
2; The composition is slightly sticky.
1; The composition is sticky.

(4) Moisture retention effect:

The moisture retention effect when each cosmetic composition was used was evaluated by sensory evaluation by ten specialized panel members according to the following criteria, and an average score was obtained.

5; The composition has a considerably high moisture retention effect.
4; The composition has a moisture retention effect.
3; Cannot say either.
2; The composition has a little moisture retention effect.
1; The composition does not have a moisture retention effect.

[Table 1]

| | Component (% by weight) | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Comp. Ex. 1 | Comp Ex. 2 |
|---|---|---|---|---|---|---|---|---|---|---|---|---|---|---|
| A | Laurylamine | | | | | 1 | | | | | | | | |
| A | Oleylamine | | 3 | | | | | | | | | | | |
| A | Stearylamine | | | | | | 2.5 | | | | | | | |
| A | Phytosphingosine | 12 | | | | | | | | | | | | |
| A | Hydroxyethylisostearyloxyisopropanolamine | | | 20 | | | | 6 | | | 0.1 | 9 | | |
| A | Hydroxyethylstearyloxyisopropanolamine | | | | 10 | | | | | | | | | |
| A | Distearylamine | | | | | | | | | | | | | 2 |
| A | Dimethylstearylamine | | | | | | | | 9 | | | | | |
| A | Stearyltrimethylammonium chloride | | | | | | | | | 1.5 | | | | |
| | Isostearic acid | | | | | | | | | | | | 1 | |
| B | Carbomer (CARBOPOL 981, Lubrizol Advanced Materials) | 4 | | | | | | | | | | | | |
| B | Carbomer (CARBOPOL 980, Lubrizol Advanced Materials | | | | 5 | | | | | | | | | |
| B | (Acrylates/alkyl acrylate (C10-30)) crosspolymer (CARBOPOL ETD2020, Lubrizol Advanced Materials) | | | | | 1 | | | | | | | | |
| B | Carbomer (CARBOPOL ULTREZ 10, Lubrizol Advanced Materials) | | | | | | 0.35 | | | | | | | |
| B | Carbomer (CARBOPOL ULTREZ 20, Lubrizol Advanced Materials) | | | 10 | | | | | | | | | | |
| B | Carbomer (CARBOPOL ULTREZ 21, Lubrizol Advanced Materials) | | | | | | | | | 1 | | | | |
| B | Acrylates copolymer (CARBOPOL AQUA-SF1, Lubrizol Advanced Materials) | | | | | | | 0.6 | | | | | | |

EP 2 520 277 A1

| | Component (% by weight) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Comp. Ex. 1 | Comp Ex. 2 |
|---|---|---|---|---|---|---|---|---|---|---|---|---|---|---|---|
| B | (Alkyl acrylate/Steareth-20 methacrylate) copolymer (ACULYN 22, manufactured by Rohm & Haas) | | | | | | | | | | 1 | | | 1 | |
| B | (Acrylic acid/alkyl acrylate (C10-30)) crosspolymer (PEMULEN TR-1, Lubrizol Advanced Materials) | | | | | | | | | | | 0.1 | | | |
| B | (Acrylic acid/alkyl acrylate (C10-30)) crosspolymer (PEMULEN TR-2, Lubrizol Advanced Materials) | | | 0.6 | | | | | | | | | | | |
| B | Genupectin (GENU pHresh DF, Sanshosha) | | | | | | | | | | | | 0.1 | | |
| | Guar gum | | | | | | | | | | | | | | 1 |
| | Vaseline | | | | | | | | | | | | | | |
| C | Water | Water 1 | 4 | 0.6 | 10 | 5 | 1 | 0.35 | 0.6 | 1 | 1 | 0.1 | 0.1 | 98 | 97 |
| | | Water 2 | 80 | 95.8 | 60 | 80 | 97 | 96.8 | 92.8 | 89 | 96.5 | 99.7 | 90.8 | | |
| | Formation of liquid crystal | | O | O | O | O | O | O | O | O | O | O | O | × | × |
| | Occluding effect | | 43 | 15 | 54 | 46 | 17 | 15 | 27 | 33 | 16 | 11 | 34 | 6 | 8 |
| | Feel upon use (absence of tackiness) | | 4.1 | 4.5 | 3.9 | 4.1 | 4.8 | 4.6 | 4.4 | 4.2 | 4.7 | 5 | 4.3 | 4.8 | 4.7 |
| | Moisture retention effect | | 4.9 | 4.3 | 5 | 4.9 | 4.5 | 4.3 | 4.7 | 4.8 | 4.6 | 4.1 | 4.8 | 3.9 | 3.9 |
| | A/B | | 3.00 | 5.00 | 2.00 | 2.00 | 1.00 | 7.14 | 10.00 | 9.00 | 1.50 | 1.00 | 90 | - | - |

EP 2 520 277 A1

Examples 12 to 15

The aqueous cosmetic compositions having the compositions shown in Table 2 were produced, and the formation of a liquid crystal, occluding effect, feeling upon use and moisture retention effect were evaluated in similar manners to those in Examples 1 to 11. The results are shown collectively in Table 2.

(Production method)

The components (A), (B) and (water 1) were mixed, the mixture was homogenized by heating to a temperature equal to the melting point of the component (A) (80°C) or more, the component (B) was neutralized with the component (A) to form a salt, thereby a liquid crystal structure was formed, the liquid crystal structure was homogenized by mixing with the component (D), water 2 was further added thereto to disperse the liquid crystal structure, thereby an aqueous cosmetic composition was obtained.

[Table 2]

| | Component (% by weight) | | Ex. 12 | Ex. 13 | Ex. 14 | Ex. 15 |
|---|---|---|---|---|---|---|
| A | Hydroxyethylisostearyloxyisopropanolamine | | 20 | 20 | 20 | 20 |
| B | Carbomer (CARBOPOL ULTREZ20, Lubrizol Advanced Materials) | | 10 | 10 | 10 | 10 |
| C | Water 1 | | 10 | 10 | 10 | 10 |
| | Water 2 | | 59.9 | 30 | 10 | 59.99 |
| D | Liquid isoparaffin | | 0.1 | 30 | 50 | 0.01 |
| | Total | | 100 | 100 | 100 | 100 |
| | Formation of liquid crystal | | O | O | O | O |
| | Occluding effect | | 55 | 78 | 91 | 54 |
| | Feel upon use (absence of stickiness) | | 3.9 | 4 | 4 | 3.9 |
| | Moisture retention effect | | 5 | 5 | 5 | 5 |
| | A/B | | 2 | 2 | 2 | 2 |
| | C1/(A+B) | | 0.33 | 0.33 | 0.33 | 0.33 |

**Claims**

1.   A method for producing an aqueous cosmetic composition comprising the following components (A), (B) and (C) :

(A) a primary amine, a secondary amine, a tertiary amine or a quaternary amine having 12 to 60 carbons in total,
(B) an anionic polymer having a carboxyl group, and
(C) water, and, comprising:

a liquid crystal constituted by the components (A), (B) and (C), obtained by mixing the components (A), (B) and (C); heating the mixture to a temperature equal to or higher than the melting point of the component (A) to homogenize the mixture; and neutralizing the component (B) with the component (A) to form a salt.

2.   The method for producing an aqueous cosmetic composition according to claim 1, wherein the weight ratio of the component (A) and component (B) is (A)/(B) = 1 to 90.

3.   The method for producing an aqueous cosmetic composition according to claim 1, comprising mixing the component (A), component (B) and a part of the component (C), heating the mixture to a temperature equal to or higher than the melting point of the component (A) and neutralizing the component (B) with the component (A) to form a salt, thereby forming a liquid crystal constituted by the components (A), (B) and (C), and adding the residual water thereto.

4.   The method for producing an aqueous cosmetic composition according to claim 3, wherein the weight ratio of the component (A), the component (B) and a part of water (C') that is added initially is (C')/(A)+(B) = 0.01 to 0.5.

5. The method for producing an aqueous cosmetic composition according to claim 3, comprising mixing the component (A), component (B) and a part of the component (C), heating the mixture to a temperature equal to or higher than the melting point of the component (A) and neutralizing the component (B) with the component (A) to form a salt, thereby forming a liquid crystal constituted by the components (A), (B) and (C), followed by homogenizing the liquid crystal by mixing with (D) an oil agent that is in a liquid form at 25°C, and further adding the residual water thereto.

6. The method for producing an aqueous cosmetic composition according to claim 1, wherein the component (B) is an anionic polymer containing acrylic acid or methacrylic acid as a monomer.

7. An aqueous cosmetic composition, being obtained by the production method according to any one of claims 1 to 6.

## INTERNATIONAL SEARCH REPORT

| International application No. |
| --- |
| PCT/JP2010/073574 |

A. CLASSIFICATION OF SUBJECT MATTER
*A61K8/81*(2006.01)i, *A61K8/02*(2006.01)i, *A61K8/41*(2006.01)i, *A61Q19/00*
(2006.01)i

According to International Patent Classification (IPC) or to both national classification and IPC

B. FIELDS SEARCHED

Minimum documentation searched (classification system followed by classification symbols)
A61K8/81, A61K8/02, A61K8/41, A61Q19/00

Documentation searched other than minimum documentation to the extent that such documents are included in the fields searched
Jitsuyo Shinan Koho 1922-1996 Jitsuyo Shinan Toroku Koho 1996-2011
Kokai Jitsuyo Shinan Koho 1971-2011 Toroku Jitsuyo Shinan Koho 1994-2011

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
| --- | --- | --- |
| A | JP 2000-513745 A  (Gist-Brocades, B.V.),<br>17 October 2000 (17.10.2000),<br>example 6<br>& US 6147118 A          & EP 914075 A2<br>& WO 1998/049999 A2      & KR 10-2000-0022503 A | 1-7 |
| A | JP 7-187963 A  (Kao Corp.),<br>25 July 1995 (25.07.1995),<br>example 3<br>(Family: none) | 1-7 |
| A | JP 8-217648 A  (Kao Corp.),<br>27 August 1996 (27.08.1996),<br>claim 3; example 5<br>(Family: none) | 1-7 |

| ☒ | Further documents are listed in the continuation of Box C. | ☐ | See patent family annex. |
| --- | --- | --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 08 March, 2011 (08.03.11) | 29 March, 2011 (29.03.11) |

| Name and mailing address of the ISA/<br>Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2010/073574

C (Continuation).   DOCUMENTS CONSIDERED TO BE RELEVANT

| Category* | Citation of document, with indication, where appropriate, of the relevant passages | Relevant to claim No. |
|---|---|---|
| A | JP 11-335563 A  (Shin-Etsu Chemical Co., Ltd.),<br>07 December 1999 (07.12.1999),<br>example 18<br>& US 6290942 B1 | 1-7 |
| A | JP 2000-256188 A  (Nikko Chemicals Co., Ltd.),<br>19 September 2000 (19.09.2000),<br>claim 1; examples 5, 6<br>(Family: none) | 1-7 |
| A | JP 60-130508 A (Lion Corp.),<br>12 July 1985 (12.07.1985),<br>comparative examples 4, 5<br>(Family: none) | 1-7 |
| A | JP 61-229814 A  (Kanebo, Ltd.),<br>14 October 1986 (14.10.1986),<br>claim 1; example 4<br>(Family: none) | 1-7 |

Form PCT/ISA/210 (continuation of second sheet) (July 2009)

**INTERNATIONAL SEARCH REPORT**

International application No.

PCT/JP2010/073574

Claim 1 includes all of aqueous cosmetics described in claim 1 of the present application, each of which comprises an liquid crystal comprising an amine, an anion polymer and water. However, those aqueous cosmetics which are disclosed in the meaning within PCT Article 5 are only aqueous cosmetics in which a primary, secondary, tertiary or quaternary amine having an alkyl or alkenyl group having 12 or more carbon atoms or a sphingosine which are disclosed in paragraphs [0009]-[0010] and table 1 in the description is used as the amine. Therefore, claim 1 is not supported in the meaning within PCT Article 6.

Such being the case, the search was carried out on the scopes relating to the above-stated aqueous cosmetics.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001072581 A
- JP 2003026608 A